# DEMANDE DE BREVET EUROPEEN

(11) **EP 1 520 601 A2**
(43) Date de publication de la demande: **06.04.2005**
(21) Numéro de dépôt: 04370013.7
(22) Date de dépôt: 04.05.2004
(51) Int. Cl.: A61M 25/02, A61F 13/02

(54) **Support de drain adhésif auto décollable**

(30) Priorité: 02.10.2003 FR 0311570
(71) Demandeur: L'Echevin, Patrick, 62530 Hersin-Coupigny (FR); Berqué, Emmanuel, 59850 Nieppe (FR)
(72) Inventeur: L'Echevin, Patrick, 62530 Hersin-Coupigny (FR); Berqué, Emmanuel, 59850 Nieppe (FR); Dechypere, Dominique, 59800 Lille (FR)

(57) **Abrégé**

Support de drain adhésif auto décollable constitué d'un socle (1) muni d'un adhésif (2) pour le coller contre la peau (3) d'un patient pour maintenir en place un drain (9) sur son guide (4) caractérisé en ce qu'il est pourvu d'un moyen mécanique de levier réalisé manuellement entre l'adhésif (2) et la peau du patient (3) pour les désolidariser sans traction sur ledit support de drain.

L'invention se rapporte au domaine technique des drains médicaux et notamment aux drains dits de « Redon » passant par un orifice artificiel du corps d'un patient.

## Description

L'invention se rapporte au domaine technique des drains médicaux et notamment aux drains dits de « Redon » passant par un orifice artificiel du corps d'un patient.

Le domaine de la chirurgie ou de la réanimation fait très souvent appel au drainage afin d'évacuer du corps diverses substances. En exemple, le drainage peut intervenir en chirurgie des membres ou lors de la mise en place de prothèses (hanche, genoux, etc.) pour mettre en communication l'intérieur des cavités avec un système d'aspiration pour évacuer tout épanchement de substances.

Les drains médicaux utilisés et, notamment les drains dits de « Redon » sont classiquement fixés par une suture filaire à la peau du patient pour assurer leur maintien, un pansement étant alors disposé autour du drain.

Ce mode de fixation pose en fait de nombreux problèmes.

En effet, la fixation par suture est douloureuse pour le patient et le fait d'exercer une traction accentue cette sensation et peut être source d'infection.

De plus le drain a tendance à se plier à proximité de l'orifice pratiqué dans le corps du patient sous l'effet de traction possible au raccordement du système d'aspiration.

Enfin, la présence des pansements rend difficile l'accès au drain pour vérifier sa position ou encore la présence d'éventuelles sécrétions.

Cette problématique est accentuée par le fait qu'il faille parfois, quelques jours après l'opération, retirer le drain progressivement en deux ou trois étapes.

A chaque fois, il faudra donc refaire les pansements et couper le fil de suture pour éventuellement le fixer à la peau par une nouvelle suture.

Ces manipulations sont douloureuses pour le patient, constituent un risque d'infection au niveau de l'orifice cutané, et nécessitent un travail important pour le personnel soignant. Elles sont également coûteuses tant en main d'oeuvre qu'en produits utilisés.

Divers dispositifs ont été mis au point pour maintenir de tels drains.

On peut notamment citer le document FR 2707175 décrivant un dispositif réalisé en deux éléments séparables pour fixer le drain au corps du patient au moyen d'une zone adhésive ou encore le document WO 9825661 qui maintient également les drains dans une position déterminée.

Aucun de ces dispositifs ne décrit une particularité lors de l'enlèvement des supports de drains adhésifs de la peau du patient.

En effet, la dernière action exercée sur le support de drain est son propre décollage de la surface de la peau du patient.

Cette opération délicate est particulièrement douloureuse pour le patient car la partie adhésive doit effectivement résister suffisamment pour supporter par sa force de collage les diverses tractions exercées sur le drain pendant plusieurs jours puis être ensuite décollée de cette même peau.

De fait, l'adhésif résistant à son propre décollage produit localement, à proximité immédiate ou au niveau même de l'incision, un effet de traction sur la peau qui peut se traduire par une désunion importante de la fermeture cutanée.

Cette désunion par excès de déformation entraîne bien évidemment une douleur importante et une potentielle ré-intervention au niveau de la cicatrice.

La présente invention a pour but de remédier à ces inconvénients en proposant un dispositif de support de drains adhésif auto-décollable disposé classiquement par adhésif contre la peau d'un patient pour maintenir mécaniquement le drain et ayant la particularité de pouvoir se décoller de ladite peau par un moyen autonome et manuel pour ainsi endiguer la douleur dûe au décollage dudit adhésif.

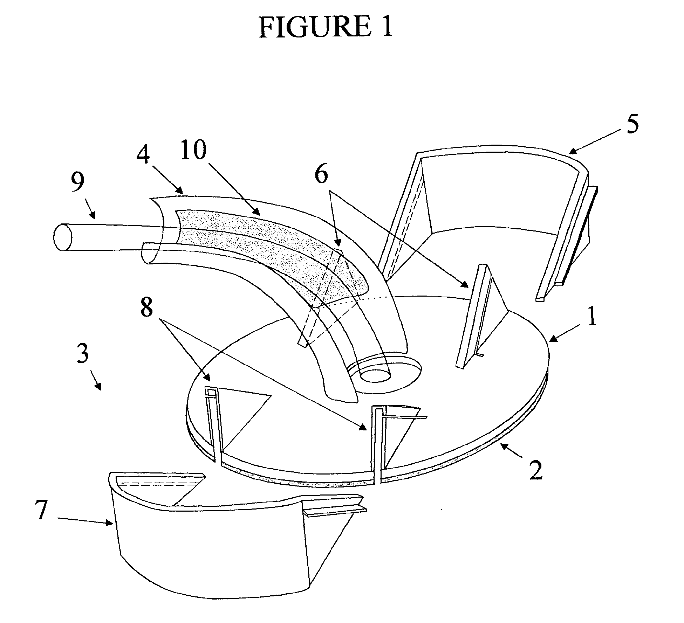

Pour ce faire et suivant la figure 1, le support de drain adhésif auto décollable constitué d'un socle (1) muni d'un adhésif (2) pour le coller contre la peau (3) d'un patient pour maintenir en place un drain (9) sur son guide (4) **est caractérisé en ce qu'il** est pourvu d'un moyen mécanique de levier réalisé manuellement entre l'adhésif (2) et la peau du patient (3) pour les désolidariser sans traction sur ledit support de drain.

En effet, le socle du dispositif selon l'invention comprend, sur sa face en contact avec la peau, un adhésif afin de pouvoir le coller sur la peau du patient.

Le socle comprend également un guide en son centre pour que le drain se fixe mécaniquement contre ce dernier pour assurer de façon efficace le maintien du drain sur le patient.

De façon particulière, le socle est pourvu d'un moyen mécanique créant un effet manuel de levier entre l'adhésif et la peau du patient afin de ne pas devoir tirer fortement sur le support de drain pour le décoller de la peau du patient et par là même endiguer les effets de douleurs précités.

Cet effet de levier est donc produit lors de l'enlèvement du support de drain par une action manuelle qui sépare mécaniquement l'adhésif de la peau du patient et ceci sans devoir appliquer de traction sur le support de drain pour le décoller.

Les effets de ce décollage par action de levier sont particulièrement intéressants car ils n'engendrent pas d'arrachement par traction extrême à proximité immédiate de l'incision et de fait n'engendrent pas d'intervention supplémentaire sur la cicatrice.

Pour ne pas engendrer de traction sur le support de drain pour son décollage, l'effet manuel de levier doit s'exercer de façon tangentielle à la surface de la peau.

De fait, le dispositif selon l'invention est **caractérisé en ce que** le moyen mécanique de levier réalisé entre l'adhésif (2) et la peau du patient (3) s'exerce par pression manuelle de chaque côté du socle (1) tangentiellement à la surface de la peau.

En outre, l'effet de levier manuel peut s'exercer de deux façons, soit unilatérale soit bilatérale, c'est-à-dire que le décollage de l'adhésif par rapport à la peau peut s'exercer d'un côté du socle jusqu'à son extrémité opposée ou des deux côtés du socle à la fois.

De façon unilatérale, le socle bascule en se décollant de la peau; de façon bilatérale le socle se soulève en se décollant de la peau des deux côtés en même temps.

De fait, le dispositif selon l'invention est **caractérisé en ce que** le moyen mécanique de levier réalisé entre l'adhésif (2) et la peau du patient (3) s'exerce de façon unilatérale ou bilatérale simultanée de chaque côté du socle (1).

Cette pression manuelle tangentielle au socle et donc à la peau entraîne la mise en place de glissières située sur le socle pour recevoir un ensemble de coins opposés servant à produire l'effet de levier précité.

Pour ce faire et suivant les figure 1 à 4, le dispositif selon l'invention est **caractérisé en ce que** le moyen mécanique de levier est réalisé par la coulisse d'au moins un support de coins (5) mobile dans au moins un support de glissière (6) solidaire du socle (1).

Le ou les supports de coins mobiles sont donc insérés dans le ou les supports de glissières solidaires du socle.

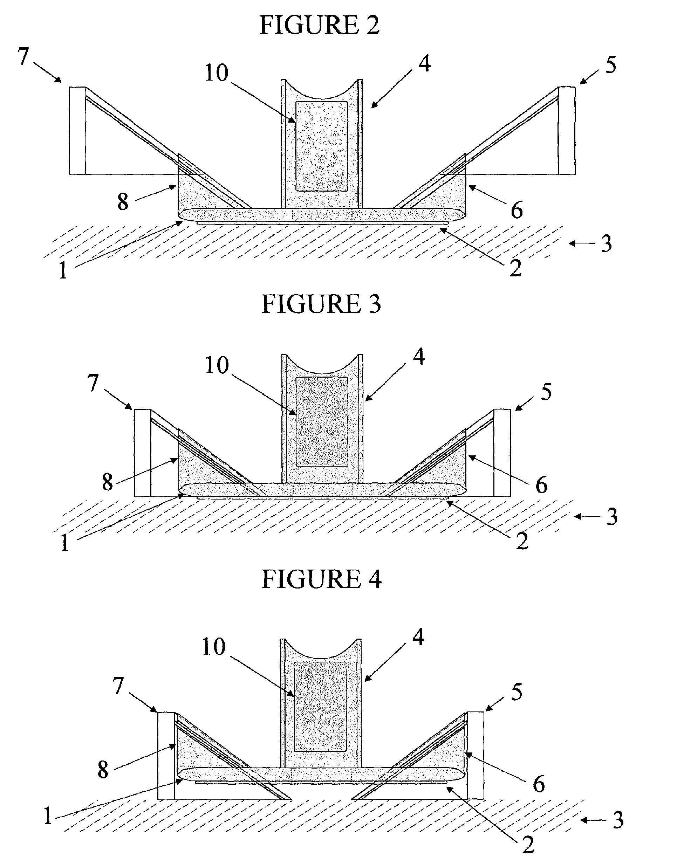

En exemple d'application bilatérale et selon les figures 2 et 3, les supports de coins (5) et (7) coulissent dans les supports de glissières (6) et (8) du socle (1).

Une pression manuelle opposée sur ces supports de coins entraîne donc leur coulissage simultané jusqu'à ce qu'ils entrent en contact avec la peau du patient, au niveau du socle, à ce stade le support de drain est toujours collé à la peau du patient.

En exemple d'application bilatérale et selon la figure 4, la pression manuelle continue de s'exercer simultanément sur les supports de coins ce qui produit un appui et une poussée entre le socle équipé de son adhésif et la peau du patient. Cette poussée engendre alors un effet de levier qui désolidarise l'adhésif de la peau du patient et décolle ainsi le support de drain selon l'invention.

Ce décollage peut également aider à la sortie du drain fixé sur le guide du socle. En effet, le drain est maintenu sur son guide par la disposition d'un adhésif dans la gorge du guide.

Il est à noter que cet adhésif peut être réutilisable afin de procéder à l'extraction du drain en plusieurs fois.

De fait, le dispositif selon l'invention est **caractérisé en ce que** le guide (4) solidaire du socle (1) et disposé en son centre possède un adhésif (10) réutilisable pour sécuriser le drain (9) par collage.

De cette manière, le support de drain selon l'invention permet d'insérer le drain en son milieu pour être descendu et collé contre la peau du patient puis, au bout d'un certain temps, permet d'être enlevé sans traction de la peau du patient et donc sans douleur.

## Revendications

1. Support de drain adhésif auto décollable constitué d'un socle (1) muni d'un adhésif (2) pour le coller contre la peau (3) d'un patient pour maintenir en place un drain (9) sur son guide (4) **caractérisé en ce qu'**il est pourvu d'un moyen mécanique de levier réalisé manuellement entre l'adhésif (2) et la peau du patient (3) pour les désolidariser sans traction sur ledit support de drain.

2. Support de drain adhésif auto décollable suivant la revendication 1, **caractérisé en ce que** le moyen mécanique de levier réalisé entre l'adhésif (2) et la peau du patient (3) s'exerce par pression manuelle de chaque côté du socle (1) tangentiellement à la surface de la peau.

3. Support de drain adhésif auto décollable suivant la revendication 1 ou 2, **caractérisé en ce que** le moyen mécanique de levier réalisé entre l'adhésif (2) et la peau du patient (3) s'exerce de façon unilatérale ou bilatérale simultanée de chaque côté du socle (1).

4. Support de drain adhésif auto décollable suivant l'une des revendications précédentes, **caractérisé en ce que** le moyen mécanique de levier est réalisé par la coulisse d'au moins un support de coins (5) mobile dans au moins un support de glissière (6) solidaire du socle (1).

5. Support de drain adhésif auto décollable suivant l'une des revendications précédentes, **caractérisé en ce que** le guide (4) solidaire du socle (1) et disposé en son centre possède un adhésif (10) réutilisable pour sécuriser le drain (9) par collage.
